Europäisches Patentamt

European Patent Office

Office européen des brevets

(19)

(11) Publication number: 0 372 651
A2

## (12) EUROPEAN PATENT APPLICATION

(21) Application number: 89203072.7

(22) Date of filing: 04.12.89

(51) Int. Cl.⁵: C08F 8/44, C10M 145/00

(30) Priority: 05.12.88 GB 8828340

(43) Date of publication of application:
13.06.90 Bulletin 90/24

(84) Designated Contracting States:
BE DE ES FR GB IT NL

(71) Applicant: SHELL INTERNATIONALE
RESEARCH MAATSCHAPPIJ B.V.
Carel van Bylandtlaan 30
NL-2596 HR Den Haag(NL)

(72) Inventor: Brons, Henricus Maria Johannes
Badhuisweg 3
NL-1031 CM Amsterdam(NL)
Inventor: van Kruchten, Eugene Maria
Godfried Andre
Carel van Bylandtlaan 30
NL-2596 HR The Hague(NL)

(74) Representative: Aalbers, Onno et al
P.O. Box 302
NL-2501 CH The Hague(NL)

(54) Succinic acid derivatives and their use as lubricant additives.

(57) A basic alkaline earth metal salt of a long chain hydrocarbon substituted derivative of succinic acid in which the hydrocarbon substituent has a number average molecular weight between 120 and 5000, together with the preparation of such materials and their use as luboil additives.

EP 0 372 651 A2

# SUCCINIC ACID DERIVATIVES AND THEIR USE AS LUBRICANT ADDITIVES

The present invention relates to succinic acid derivatives, their use as dispersant additives in luboils, and to lubricating oil compositions containing such derivatives.

The use of alkaline earth metal salts of organic sulphonic acids as additives for lubricating oil compositions is well known. rnese salts have dispersant properties so that, when applied in such luboil compositions, they ensure that the insides of the engine cylinders remain clean and that deposition of carbonaceous products on pistons and in piston grooves is counteracted, thereby preventing piston-ring sticking.

It is also well-known to prepare basic (or overbased) alkaline earth metal salts of such acids. The overbasing provides an alkaline reserve which, when applied in lubricating oil compositions, reacts with and neutralises acidic compounds formed during the operation of the engine in which the composition is applied. Hence, sludge which may arise is maintained dispersed due to the dispersant property of the salt, and acids which would enhance sludge formation are neutralised.

Although such carboxylic/sulphonic acid salts have proved reasonably effective at providing dispersant properties in luboils, increasing demands on engine performance and cleanliness provide a continued need for alternative products.

It has now been found that basic salts of certain succinic acid derivatives can provide effective dispersancy, and accordingly the present invention provides a basic alkaline earth metal salt of a long-chain hydrocarbon substituted derivative of succinic acid in which the hydrocarbon substituent has a number average molecular weight between 120 and 5000.

The long-chain hydrocarbon substituent in the succinic acid derivative is preferably an oligomer or polymer derived from an olefin, and containing at least 8 carbon atoms. When the substituent is an oligomer, this may be based on from 3-12 monomer units, with higher chain lengths being regarded as polymers. The polymers may be homopolymers or copolymers of one or more olef in monomers, such copolymers including random, block and tapered copolymers. Preferred olefin monomers are those having 2 to 16 carbon atoms, preferably from 2 to 6 carbon atoms, such as ethene, propene, butenes, isobutenes, pentenes, octenes, and also diolefines such as butadiene and isoprene. If a diene is used as monomer the resulting oligomer or polymer is preferably hydrogenated to saturate at least 90%, more preferably substantially all unsaturated bonds. Conveniently the hydrocarbon substituent is a linear oligomer or polymer derived from ethylene, or a branched one derived from propene or isobutylene.

Particularly suitable oligomeric long-chain hydrocarbon substituents are the higher olefins derived from oligomerisation of ethylene (eg. as in the Shell Higher Olefins Process "SHOP"), especially those containing 14 to 18 carbon atoms, and also oligomers of propylene, such as propylene tetramer. Particularly suitable polymeric long chain hydrocarbon substituents are those derived from isobutylene.

As stated above the average number molecular weight of the long-chain hydrocarbon substituent should be from 120 to 5000, and is preferably from 150 to 1500. The number average molecular weight (Mn) can be determined by the established techniques of vapour pressure osmometry or gel permeation chromatography with calibration of the polymer. The weight average molecular weight (Mw) can also be determined by gel permeation chromatography. The quotient Mw/Mn, which is a measure indicating the width of molecular weight distribution, usually has a value from 1.5 to 4.0.

The average number of succinic groups per hydrocarbon group may be greater than 1, in which case at least some hydrocarbon moieties are connected to at least two succinic groups. Preferably the average number of succinic groups per hydrocarbon group is between 0.5 and 3.

Hydrocarbon-substituted succinic anhydrides can be prepared by known procedures. When the substituent is a polyolefin, the substituted succinic anhydride can conveniently be prepared by mixing the polyolefin, e.g. polyisobutylene, with maleic anhydride and passing chlorine through the mixture, yielding hydrochloric acid and alkenyl-substituted succinic anhydride, as described in e.g. GB-A-949,981.

Another method for the preparation of substituted succinic anhydride is described in US-A-3,172,892, namely the reaction of maleic anhydride with a halogenated, in particular chlorinated, polyolefin.

From e.g. NL-A-7412057 it is known to prepare hydrocarbon-substituted succinic anhydride by thermal reaction of a polyolefin with maleic anhydride. It is further possible to combine the methods of this Dutch application and GB-A-949,981, as is illustrated in GB-A-1,440,219 and GB-A-1,543,627. The products prepared in this way include compounds in which the alkenyl chain is connected to one or both of the alpha carbon atoms of the succinic group.

Similar processes can be used to prepare succinic derivatives bearing an oligomer substituent.

The long chain alkyl-substituted succinic acid/anhydride thus produced is then converted to a basic (overbased) salt by reaction with an alkaline earth metal compound according to any of the known overbasing procedures, e.g. as described in UK Patent Specification 786167. Thus, the succinic acid is suitably mixed in an organic solvent with at least a molar equivalent, usually a molar excess, of a hydroxide and/or oxide of the alkaline earth metal and, if a highly overbased product is required, carbon dioxide may be passed through the resulting mixture. The alkaline earth metal may be magnesium, calcium, strontium or barium, but for reasons of practical economics calcium or magnesium is normally preferred.

The mixture of succinic acid and hydroxide/oxide can be prepared in any convenient way, e.g. by mixing the alkaline earth metal hydroxide and/or oxide with a promotor, if used, and adding the blend of acids, optionally in the presence of promotor or an organic solvent, to the resulting mixture.

It is preferred to introduce a promotor, preferably an oxygen-containing organic solvent and optionally water. Suitable solvents include $C_{1-6}$ alcohols, polyhydric alcohols such us glycol, propylene glycol, glycerol or 1,3-dihydroxypropane, ethers such as $C_{1-4}$ monoethers of glycol or propylene glycol, diisopropyl ether, 1,3- or 1,4-dioxane, or 1,3-dioxolane. Preferably the promotor is a $C_{1-6}$ alcohol, in particular methanol. It will be appreciated that in industrial processes use may be made of technical solvents, and that the use of technically pure promotors, such as methanol, might introduce the presence of water. Hence, in such cases addition of water per se is not required since its addition is made implicitly by the addition of the promotor. In general, the water content is preferably adjusted such that the percentage of water in the mixture amounts to 0 to 10%w, based on the total liquids.

Generally, it is convenient to mix the substituted succinic acid and the alkaline earth metal hydroxide and/or oxide in an organic solvent and subsequently to add the promotor. The organic solvent is suitably a hydrocarbon such as an aromatic hydrocarbon or a hydrocarbon fraction rich in aromatics, such as gasoline, with compounds such as benzene, toluene or, especially, xylene being preferred. The amount of the solvent is not critical. Promotor: solvent volume ratios up to 1:1 can suitably be applied, preference being given to ratios in the range from 0.1:1 to 0.6:1.

The concentration of the substituted succinic acid in the solvent or solvent mixture can vary within wide limits. Suitably the acid equivalent concentration is from 0.01. to 1 molar equivalent/kilogram, preferably from 0.1 to 0.8 based on the combined weight of acid and hydrocarbon solvent.

The amount of alkaline earth metal to be added should be at least 1 equivalent, and is normally a molar excess so that the subsequent carbon dioxide supply results in very basic compounds. The basicity of the products is generally expressed as the basicity index (BI), which is defined as the equivalents ratio of the total of alkaline earth metal to the total of organic acids. For a high BI the amount of alkaline earth metal hydroxide and/or oxide is preferably from 10 to 25 equivalents per equivalent acid.

The temperature at which the components are mixed is not critical, and may be ambient temperature or elevated temperature. Suitable temperatures include 15-150°C. If carbon dioxide is then introduced, the temperature is advantageously from 15 to 150°C, preferably from 30 to 110°C. In order to obtain the elevated temperature it may be necessary to employ elevated pressures, since the desired reaction temperature may be above the atmospheric reflux temperature of the reaction mixture. Suitable pressures include between 1 and 15 bar abs (1 and $15 \times 10^5$ Pa). Higher pressures are possible, but merely add to the costs of the process. The rate at which the carbon dioxide is introduced is advantageously from 0.05 to 1.0 equivalent carbon dioxide per equivalent acid per minute. The carbon dioxide introduction is conveniently carried out by passing carbon dioxide, or a mixture of carbon dioxide with an inert gas, such as air or nitrogen, through the reaction mixture under slightly higher pressure than the pressure prevailing in the reaction mixture. Higher pressures may be employed. Carbon dioxide will be absorbed in the reaction mixture and will react with the alkaline earth metal compounds present therein forming a basic complex salt of the organic acid salt and carbonate, hydroxide and/or oxide. The amount of carbon dioxide to be taken up is to a certain extent dependent on the amount of alkaline earth metal present. Suitably the relative amount of carbon dioxide is somewhat less than the relative amount of alkaline earth metal hydroxide or oxide.

Preferably the introduction of carbon dioxide is stopped after 0.5 to 0.9 equivalent carbon dioxide per equivalent alkaline earth metal has been taken up, which suitably corresponds with 5 to 23 equivalent carbon dioxide per equivalent acid for high BI's mentioned above.

It has been found that an ageing period between introduction of carbon dioxide and subsequent purification can be advantageous, since it increases the BI of the resulting basic salt. Such an ageing period is suitably at least 15 min, with practical and/or economical factors generally requiring a maximum period below 20 hours. Preferably the ageing period is from 1 to 4 hours.

The mixture resulting from the reaction may be worked up by any method known in the art. It may be subjected to a centrifuging treatment to remove solids such as unreacted alkaline earth metal hydroxide and/or oxide and/or non-colloidal alkaline earth metal carbonate, if any. The resulting solution may then be subjected to a liquid-phase separation. One liquid phase can be an aqueous layer which may contain the promotor when it is used, the other one is the hydrocarbon solvent plus the basic salts dispersed therein. It is also possible to reverse the above operations.

The present process can be used for the preparation of basic salts having a wide variety of basicity indices. It can, therefore, be used to prepare basic salts having a relatively low BI e.g. from 1 to 10, but is also suitable for preparing basic salts having a basicity index from 10 to 20.

The process described is a one-step process, but it is also possible to operate as a two-step process, such as the two-step process described in Applicants' EP-A-248465.

The basic salts are excellent dispersant additives for oils. The present invention therefore also provides lubricating oil compositions comprising a major amount of a base oil and a minor amount of an overbased alkaline earth metal salt of substituted succinic acid of the invention. Preferably the base oils is a lubricating base oil, and conveniently constitutes more than 50%w of the composition. It can be selected from mineral lubricating oils of varying viscosities, but it also may be a synthetic lubricant, such as ester-type lubricant or a polyolefin-type fluid, or a vegetable oil, or a grease.

Fuel compositions which are used in marine diesel engines usually contain some sulphur compounds. To neutralize the acidic compounds formed from these sulphur compounds a relatively high concentration of the basic salt is usually employed in lubricating oil compositions for such marine applications, suitably being from 5 to 30%w of overbased alkaline earth metal succinate. Lubricating oil compositions for road engines may contain lower concentrations. The amount of overbased alkaline earth metal succinate in such lubricating oil compositions is preferably from 0.01 to 5%w, in particular from 0.1 to 4.0%w.

Fuels, such as gasoline, kerosine, diesel, fuel sand gas oils, can also contain the above basic salts. The amount of these salts is similar to that in road engine lubricating oil compositions or lower; conveniently the amount is from 0.001 to 5%w, in particular from 0.01 to 1.0%w.

The lubricating oil composition can be prepared by mixing a concentrate containing up to 60%w of a basic salt as described above in a lubricating oil, with a lubricating base oil to give the desired concentration. The lubricating oil may be the same as the one indicated above as a suitable hydrocarbon solvent. The concentrate may conveniently contain a stabiliser, which can be selected from a variety of organic compounds, such as those described in British patent specification No. 818,325. These compounds include mono- or polyhydric alcohols, alkyl amines and alkyl phenols.

The lubricating oil compositions may further contain a number of other additives, such as antioxidants, foam inhibitors, corrosion inhibitors, viscosity index improvers, and pour point depressants as can be established by a person skilled in the art.

The invention will be illustrated by means of the following Examples.

Example 1

The reaction product of polyisobutylene and maleic anhydride (PIB Mol wt 950; MALA: PIB mol. ratio 1:1; acid content of 1.94 eq./kg) was dissolved in xylene, neutralised with lime (calcium hydroxide), and, following addition of methanol, carbonated by the passage of carbon dioxide gas. The reaction was carried out in a 2 litre baffled glass SFS autoclave equipped with a 1cm recycle loop driven by a MAAG gear pump at c. 100 l/hr.. Carbon dioxide gas was introduced at the bottom of the reactor, the flow rate being measured with a Brooks mass-flow meter, and the reactor was stirred with a Rushton turbine stirrer at 2.5 kw/m$^3$. The reaction products were separated by centrifugation, followed by phase separation and solvent switch from xylene to "HVI60" luboil in a rotary evaporator.

This reaction was carried out at different levels of neutralisation to give products of varied B.I., and the viscosity characteristics and stability of these products evaluated. Details of the actual reactant proportions and of the product properties are set out in Table I below. That Table includes also the results of blend stability determinations carried out after dilution with a mixture of hydrotreated luboils to give a total base number of 70 mg KOH/g. These solutions were stored in tubes at ambient temperature and after 2 and 7 days they were visually assessed for amount of deposits and for brightness. The amount of deposits was expressed in % by volume. The brightness was classified as "bright" (B), "slightly hazy" (sH), "hazy" (H) and "muddy" (M). By "muddy" is understood the situation that the whole test tube is cloudy and that no visual assessment of the amount of deposits is possible.

The results of the measurements are shown in the Table I below.

## TABLE I

| Product | A | B | C |
|---|---|---|---|
| Acid conc in xylene eq/kg | 0.3 | 0.3 | 0.3 |
| methanol, %w on total liquid | 11.5 | 11.5 | 11.5 |
| Ca/acid intake, eq/eq | 19.0 | 13.0 | 4.0 |
| $CO_2$/acid, eq/eq | 12.4 | 7.0 | 2.0 |
| Neutralization | | | |
| time, h | 1 | 1 | 1 |
| temp, °C | 50 | 50 | 50 |

EP 0 372 651 A2

### TABLE I (Continued)

| Product | A | B | C |
|---|---|---|---|
| **Carbonation** | | | |
| $CO_2$ addition time, min (0.12eq/eq acid min) | 103 | 58 | 17 |
| temp, $^{\circ}C$ | 60 | 60 | 60 |
| **Final product in "HVI 60" oil** | | | |
| B.I. | 14.0 | 7.2 | 3.4 |

## TABLE I (Continued)

| Product | A | | B | | C | |
|---|---|---|---|---|---|---|
| Viscosity, mm2/s at 100°C | %w Ca | visco | %w Ca | visco | %w Ca | visco |
| | 11.00 | 41400 | 4.30 | 35 | 2.5 | 614 |
| | 9.40 | 4273 | | | | |
| | 9.05 | 1020 | | | | |
| | 8.00 | 476 | | | | |
| | 7.82 | 172 | | | | |
| | 5.75 | 41 | | | | |
| | 0.00 | 5 | | | | |
| Blend stability SMS 2580 25°C and 140°C 2 days and 7 days | O/B (70 TBN) | | O/B (70 TBN) | | O/B (70 TBN) | |

EP 0 372 651 A2

Example 2

Products similar to those of Example 1 were prepared, except that the PIB/MALA reactant was partially or wholly replaced by a $C_{18}$ olefin derived from oligomerisation of ethylene. The detailed reaction conditions were as follows:-

acid conc. in xylene 0.5 meq/g
acid/Ca(OH)$_2$/CO$_2$ : 1/4/1.5 eq/eq.
30% m methanol (on liquid intake)
CO$_2$ dosing rate : 0.082 meq/meq min.
The calcium content, B.I. and blend stability determined for these products is set out in Table 2 below.

TABLE 2

| Product | D | E |
|---|---|---|
| Long-chain hydrocarbon | | |
| PIB % eq/eq | 25 | - |
| $C_{18}$ "SHOP" % eq/eq | 75 | 100 |
| Blend in "HVI 60" oil | | |
| % Ca | 6.03 | 5.99 |
| BI | 2.84 | 2.73 |
| Stability 2d | O/B | O/B |
| 7d | O/B | O/B |
| 14d | O/B | O/B |

**Claims**

1. A basic alkaline earth metal salt of a long chain hydrocarbon substituted derivative of succinic acid in which the hydrocarbon substituent has a number average molecular weight between 120 and 5000.

2. Basic salt as claimed in claim 1 wherein the long-chain hydrocarbon substituent in the succinic acid is an oligomer or polymer containing at least 8 carbon atoms derived from an olefin.

3. Basic salt as claimed in claim 2 wherein the long-chain hydrocarbon substituent is an oligomer obtained from ethylene, propylene or butylene oligomerisation, or a polyisobutylene.

4. Basic salt is claimed in any one of claims 1, 2 or 3 wherein the number average molecular weight of the long-chain hydrocarbon substituent is from 150 to 1500.

5. Basic salt as claimed in any one of claims 1, 2, 3 or 4 wherein the average number of succinic groups per hydrocarbon group is between 0.5 and 3.

6. Basic salt as claimed in any one of the preceding claims wherein the alkaline earth metal is magnesium or calcium.

7. Process for the preparation of a basic alkaline earth metal salt as defined in claim 1, which comprises reacting an olefin polymer or oligomer with maleic anhydride, followed by the addition of at least a molar equivalent amount of an alkaline earth metal oxide or hydroxide, and optionally introducing carbon dioxide into the mixture obtained.

8. Process as claimed in claim 7 wherein the reaction is carried out in the presence of an oxygen-containing organic solvent, and optionally water, as promotor.

9. Process as claimed in claim 7 or 8 wherein carbon dioxide is introduced at a rate of from 0.05 to 1.0 equivalent per equivalent of acid per minute, followed by an ageing period of at least 15 minutes before final purification.

10. Oil composition comprising a major amount of a lubricating base oil and a minor amount of a basic

8

alkaline earth metal salt as claimed in any one of claims 1 to 6.